(19) **Europäisches Patentamt / European Patent Office / Office européen des brevets**

(11) **EP 1 788 000 A1**

(12) **EUROPEAN PATENT APPLICATION**
published in accordance with Art. 158(3) EPC

(43) Date of publication:
**23.05.2007 Bulletin 2007/21**

(51) Int Cl.:
*C08B 31/04* (2006.01)  *A61K 31/718* (2006.01)
*A61P 1/02* (2006.01)  *A61P 3/04* (2006.01)
*A61P 3/06* (2006.01)  *A61P 3/10* (2006.01)
*A61P 7/02* (2006.01)  *A61P 9/04* (2006.01)
*A61P 9/10* (2006.01)  *A61P 9/12* (2006.01)
*A61P 13/12* (2006.01)  *A61P 25/00* (2006.01)
*A61P 27/12* (2006.01)  *A61P 35/00* (2006.01)
*A23L 1/30* (2006.01)

(21) Application number: **05781951.8**

(22) Date of filing: **07.09.2005**

(86) International application number:
**PCT/JP2005/016402**

(87) International publication number:
**WO 2006/028122 (16.03.2006 Gazette 2006/11)**

(84) Designated Contracting States:
**DE FR GB**

(30) Priority: **09.09.2004 JP 2004262492**

(71) Applicant: **Kao Corporation**
**Chuo-ku**
**Tokyo 103-8210 (JP)**

(72) Inventors:
• **SHIMOTOYODOME, Akira,**
**c/o Kao Corp. Research Labs.**
**Haga-gun,**
**Tochigi 3213497 (JP)**

• **SUZUKI, Junko,**
**c/o Kao Corp. Research Labs**
**Haga-gun,**
**Tochigi 3213497 (JP)**
• **SUGINO, Nanami,**
**c/o Kao Corporation Research Labs.**
**Haga-gun,**
**Tochigi 3213497 (JP)**
• **NAKAMURA, Junji,**
**c/o Kao Corporation Research Labs**
**Haga-gun,**
**Tochigi 3213497 (JP)**

(74) Representative: **HOFFMANN EITLE**
**Patent- und Rechtsanwälte**
**Arabellastrasse 4**
**81925 München (DE)**

(54) **AGENT FOR PREVENTING AND AMELIORATING OBESITY**

(57) The present invention relates to raw materials for foods, pharmaceuticals, and the like, wherein the raw materials can be effective in preventing and ameliorating various lifestyle-related diseases such as obesity and hyperlipidemia, safe, applicable in wide areas, and less deteriorative to texture; and the present invention also relates to a preventive/remedy for obesity, an agent for suppressing fat accumulation in internal organs, an agent for suppressing blood glucose level increase, or a preventive/remedy for diabetes, wherein acetylated starch or starch octenyl succinate is an active ingredient.

EP 1 788 000 A1

**Description**

Field of the Invention

The present invention relates to a preventive/remedy for obesity and diabetes, and the like.

Background of the Invention

Recently, incidence of lifestyle-related diseases such as obesity and diabetes is steadily on the rise among Japanese due to lack of exercise in addition to excessive energy intake (an increase in intake of fat and sucrose) resulted from westernized eating habit. Under such social circumstances, taking measures to prevent and ameliorate obesity and diabetes is of great importance.

For preventing and ameliorating obesity and diabetes, a typical method suggested by nutritionists is to ingest low-calorie diets or low-fat diets. In recent years, it was reported that each of water-insoluble dietary fibers such as wheat bran, water-soluble dietary fibers such as indigestible dextrin, and resistant starches such as high amylose starch has promoting action of fat excretion (for example, see Non-Patent Document 1), inhibitory action of glucose absorption (for example, see Non-Patent Document 2), reducing action of blood triglyceride (for example, see Non-Patent Document 3), or ameliorating action of glucose resistance (for example, see non-Patent Documents 4, 5, and 6). It is believed that they are effective in prevention and amelioration of obesity as well as prevention and amelioration of diabetes.

It is also believed that a sudden increase in blood lipid levels after meals promotes fat accumulation. Hence, for preventing and ameliorating obesity, an approach to suppress hyperlipidemia (an increase in blood triglyceride) after meals is also of great importance. In recent years, as safe and effective fat absorption inhibitors, xanthan gum, propylene glycol alginate (for example, see Patent Document 1), and chitosan (for example, see Patent Document 2) were reported.

The above low-calorie diets or low-fat diets can be transiently effective to a small extent in weight loss. However, ingredients composing those diets have simple flavors, so that after a long while people who have been eating them start rejecting them, resulting in difficulty in ingesting such diets for a long period. Also, in order that ingredients such as the above conventional water-insoluble dietary fibers, water-soluble dietary fibers, and resistant starches provide the above physiological effects, they should be ingested in high doses and over a long period. Also, even when such physiological effects were obtained, suppression of obesity was not confirmed. In addition, when foods and beverages were made by adding such ingredients, appearance and texture such as taste, crunchiness, and smoothness, which were expected originally from such foods and beverages, were compromised in many cases. Therefore, there were problems in that it was difficult to contain them in sufficient amounts in foods; application areas were limited; and further, it was difficult to keep ingesting such foods and beverages over a long period.

Acetylated starch is superior in freeze-thaw stability and transparency, and thus utilized in areas such as frozen noodles for improving texture and stability and frozen omelets for preventing the separation of water content (for example, see Non-Patent Document 7). Starch octenyl succinate is a starch wherein an emulsifying ability is added, and such starch has two functions, i.e., emulsification and emulsion stabilization (for example, see Non-Patent Document 7), and is used as an additive in shampoos, cosmetics such as powders, and the like.

However, it has not been noted so far that the acetylated starch or starch octenyl succinate is effective in preventing and ameliorating lifestyle-related diseases such as obesity and diabetes.

[Non-Patent Document 1] Am J Clin Nutr 1978 31 (10 Suppl) : 521-529
[Non-Patent Document 2] Endocrine Journal 1992 68 (6): 623-35
[Non-Patent Document 3] Am JClinNutr 198949 (2) : 337-44
[Non-Patent Document 4] Acta Paediatr Hung 1985 26 (1) : 75-7
[Non-Patent Document 5] J Endocrinol 1995 144 (3) : 533-8
[Non-Patent Document 6] Am J Clin Nutr 1989 49 (2) : 337-44
[Non-Patent Document 7] Toyoharu Takahashi, "Denpun Seihin No Chishiki" (in Japanese) (Translation: "Knowledge on Starch Products"), Saiwai Shobo, pp 112 & 132
[Patent Document 1] JP-A-H05-186356
[Patent Document 2] JP-A-H03-290170

Disclosure of the Invention

The present invention relates to a preventive/remedy for obesity, an agent for suppressing fat accumulation in internal organs, an agent for suppressing blood glucose level increase, and a preventive/remedy for diabetes, wherein the acetylated starch or starch octenyl succinate is an active ingredient.

The present invention also relates to use of the acetylated starch or starch octenyl succinate for preparing a preventive/remedy for obesity, an agent for suppressing fat accumulation in internal organs, an agent for suppressing blood glucose level increase, or a preventive/remedy for diabetes.

The present invention also relates to a method for preventing and ameliorating obesity, a method for suppressing fat accumulation in internal organs, a method for suppressing blood glucose level increase, or a method for preventing and ameliorating diabetes, wherein the acetylated starch or starch octenyl succinate is administered or ingested.

The present invention further relates to a food for preventing and ameliorating obesity, a food for suppressing fat accumulation in internal organs, a food for suppressing blood glucose level increase, or a food for preventing and ameliorating diabetes, wherein the acetylated starch or starch octenyl succinate is included.

Best Mode for Carrying Out the Invention

The present invention relates to providing raw materials for foods, pharmaceuticals, and the like, wherein the raw materials are not only effective in preventing and ameliorating various lifestyle-related diseases such as obesity and hyperlipidemia, safe, and applicable in wide areas, but also less deteriorative to texture.

The present inventors have been exploring for raw materials that have physical properties different from those of the conventional dietary fibers, such as resistant starches, cellulose, and indigestible dextrin and are also effective in suppressing progression of obesity and diabetes and ameliorating such diseases, and finally they have discovered that acetylated starch or starch octenyl succinate, exhibiting in a small ingesting amount various physiological actions such as obesity-suppressing action, is useful as a raw material for foods and pharmaceuticals that is effective in preventing and ameliorating lifestyle-related diseases such as obesity and diabetes.

The preventive/remedy for obesity and the like of the present invention can be effective in prevention and amelioration of the development of various lifestyle-related diseases, for example, in prevention and amelioration of obesity, prevention and amelioration of hyperlipidemia, prevention of cardiac diseases such as cardiac failure, prevention of thrombosis, prevention of colonic cancer or rectal cancer, and hence can be advantageously used as foods, pharmaceuticals, and the like. In particular, the acetylated starch or starch octenyl succinate, which is an active ingredient thereof, is derived from starch, and hence it is safe for humans. The acetylated starch or starch octenyl succinate is also easily gelatinized, and hence it is applicable in a wide range of foods and beverages and when it is added to a specially designated food for promoting better health or the like, it will compromise the original texture thereof only at a minimal level.

The acetylated starch in accordance with the present invention can be obtained by acetylating starch or processed starch by an ordinary method. Specifically, the acetylated starch can be obtained by reacting acetic anhydride or vinyl acetate on starch.

Commercially available acetylated starches such as Z-700 (derived from tapioca, acetylated, available from Nippon Starch Chemical), MT-01B (derived from tapioca, acetylated, available fromNihon Shokuhin Kako), ADIX-H (derived from waxy corn, acetylated, available from Nihon Shokuhin Kako), and Mapusu #449 (derived from waxy corn, acetylated, available from Nihon Shokuhin Kako) can also be used.

The starch octenyl succinate can be obtained by modifying starch or processed starch with octenyl succinate by an ordinary method. Specifically, the starch can be obtained by reacting octenyl succinic anhydride on starch.

Commercially available products such as Amycol Nyuka (derived from tapioca, modified with octenyl succinate, available from Nippon Starch Chemical) can also be used.

Starches as startingmaterials include, for example, waxy corn starch, corn starch, wheat starch, rice starch, glutinous rice starch, potato starch, sweet potato starch, tapioca starch, and sago starch. Preferably, the amylopectin content in such starch is 70% by weight or more, preferably 75 to 100%, and more preferably 90 to 100%, so that a gelatinized solution of such starch is highly transparent; when such starch is applied in foods and beverages, the appearance thereof is not compromised; and such starch is applicable in wide areas. Among them, waxy corn starch and tapioca starch are preferred as starting material starches.

The "acetylated starch or starch octenyl succinate" in accordance with the present invention also includes acetylated starch or starch octenyl succinate obtained by combining other processes with the above process. Processes that can be combined include esterification of acetic acid, phosphoric acid, etc., etherization such as carboxymethyl etherization of hydroxypropyl group or the like, cross-linking by use of an ordinary cross-linking agent such as a trimetaphosphate, a hexametaphosphate, phosphorus oxychloride, adipic acid, and epichlorohydrin, oxidation, acid treatment, bleaching, steam treatment, heat treatment, and enzymatic treatment. The above processes may be used solely or in combination of two or more. Among them, esterification is preferred, and phosphorylation, in particular, cross-linking by a phosphate is more preferred. As for the degree of phosphorylation, the bound phosphorus content may be from 0.0001 to 2%, preferably from 0.0001 to 0.5%, and more preferably from 0.0001 to 0.2%, in view of better texture and the like.

As the acetylated starch or starch octenyl succinate in accordance with the present invention, gelatinized acetylated starch or gelatinized starch octenyl succinate wherein the above acetylated starch or starch octenyl succinate is gelatinized by heating in water and the like can also be used. Herein, typical conditions for the heat treatment in water are, for example, that the above starch is suspended in distilled water or the like at the final concentration of from 4 to 60% by weight, which is then autoclaved at from 110 to 130 °C for from 10 to 20 min.

As for the degree of acetylation of the acetylated starch, the acetyl value (the number of acetyl groups per glucose anhydride residue in a starch) is preferably from 0.001 to 1, more preferably from 0. 005 to 0.5, and still more preferably from 0.01 to 0.1.

As the degree of modification with octenyl succinate, the octenyl succinate value (the number of octenyl succinate groups per glucose anhydride residue in a starch) is preferably from 0.001 to 1, more preferably from 0.005 to 0.5, and still more preferably from 0.01 to 0.1.

The above acetylated starch or starch octenyl succinate can be prepared by simple steps from starch relatively at a low cost and the resulting product is of high purity. The safety level of the above acetylated starch or starch octenyl succinate is also high. When used in various foods and beverages, pharmaceuticals, pet foods, or the like, the above acetylated starch or starch octenyl succinate gives more natural texture than the conventional dietary fibers or resistant starch. In addition, the above acetylated starch or starch octenyl succinate is resistant to freezing, so that advantageously defrosting does not readily cause deterioration thereof.

Moreover, as described in the Examples in the later paragraphs, first, the acetylated starch or starch octenyl succinate has the action of suppressing obesity, by which body weight and fat built up in internal organs are significantly reduced, and hence can be effective, for example, in prevention of hyperlipidemia, prevention of cardiac diseases such as cardiac failure, prevention of thrombosis, and prevention of hypertension, those ailments being caused by obesity. Second, the acetylated starch or starch octenyl succinate has the action of suppressing hyperglycemia after meals, that is, a sudden increase in blood glucose level after meals, as well as the action of suppressing an increase in stationary blood glucose levels, and hence can be effective, for example, in prevention of diabetes and related various complications such as cataract, periodontitis, diabetic nephropathy, retinopathy, and neuropathy.

Accordingly, the acetylated starch or starch octenyl succinate in accordance with the present invention can be a raw material for foods or pharmaceuticals for humans or animals, serving as a preventive/remedy for obesity, an agent for suppressing fat accumulation in internal organs, an agent for suppressing blood glucose level increase, and a preventive/remedy for diabetes (hereinafter referred to as the preventive/remedy for obesity and the like).

As for the preventive/remedy for obesity and the like in accordance with the present invention, one or more types of the acetylated starch or starch octenyl succinate per se can be solely administered to a human or an animal, and also can be ingested by the addition thereof to various foods and beverages, pharmaceuticals, pet foods, and the like. Applicable foods include foods for promoting beauty, foods for sick people, and specially designated foods for promoting better health, the conceptual targets thereof being physiological functions such as suppression of body fat accumulation and suppression of blood glucose level increase. Applicable pharmaceutical preparations include, for example, oral solid preparations such as tablets and granules, liquids for internal use, and oral liquid preparations such as syrup.

Furthermore, when an oral solid preparation is formed, an excipient, and optionally a binder, a disintegrator, a lubricant, a coloring agent, a flavoring agent, odor modifier, etc., are added to the acetylated starch or starch octenyl succinate in accordance with the present invention, and then tablets, coated tablets, granule, powder, capsules, or the like can be formed by the conventional method. Moreover, when an oral liquid preparation is formed, a flavoring agent, a buffer, a stabilizer, a flavoring agent, etc., are added, and then a liquid preparation for internal use, syrup, elixir, or the like can be formed by the conventional method.

The amount of the acetylated starch or starch octenyl succinate to be added in each of the above preparations is generally from 5 to 100% by weight, preferably from 20 to 100% by weight, and more preferably from 30 to 100% by weight.

The dosage (the effective ingesting amount) of the preventive/remedy for obesity and the like in accordance with the present invention is preferably 0.01 g/kg body weight per day or more, more preferably 0.1 g/kg body weight per day or more, and still more preferably 0.4 g/kg body weight per day or more.

[Examples]

Test Example 1

The action of suppressing obesity and the action of suppressing blood glucose level increase exhibited by the acetylated starch or starch octenyl succinate

A tapioca starch and a waxy corn starch were obtained from National Starch and Chemical. As acetylated starch or starch octenyl succinate, commercially available Amycol Nyuka (derived from tapioca, modified with octenyl succinate, from Nippon Starch Chemical), Z-700 (derived from tapioca, acetylated, fromNippon Starch Chemical), ADIX-H (derived from waxy corn, acetylated, from Nihon Shokuhin Kako), and Mapusu #449 (derived from waxy corn, acetylated, from Nihon Shokuhin Kako) were used.

The above starch was suspended in distilled water at the final concentration of 50% by weight, which then was autoclaved at 120 °C for 15 min. (steam treatment) followed by freeze-drying to prepare the gelatinized test starch.

Mice were divided into groups, in which each group consisted of 10 mice (C57BL/6J,males, 6 weeks old). The animal diets were prepared by adding each of the gelatinized starches according to the compositions shown in Table 1. The mice were fed with the above diets. After 15-week feeding, the mice were put away after the blood samples were collected, and the blood glucose level and the fat weight of the internal organs were measured. In Table 2, the mouse body weight after 15-week feeding as well as the fat weight of the internal organs and the blood glucose level after 15-week feeding are shown.

[Table 1] Diet compositions (% by weight)

|  | Low-fat diet | Test diet (high fat and high sucrose) |
| --- | --- | --- |
| Gelatinized test starch | 0% | 5% |
| Gelatinized potato starch | 66.5% | 23.5% |
| Sucrose | 0% | 13% |
| Fat | 5% | 30% |
| Casein | 20% | 20% |
| Cellulose | 4% | 4% |
| Mineral mixture | 3.5% | 3.5% |
| Vitamin mixture | 1% | 1% |

[Table 2]

| Mouse body weight and fat weight of the internal organs | | | | | | |
| --- | --- | --- | --- | --- | --- | --- |
|  | Derived from | Functional group | Cross-linking | Average weight after 15 weeks (g) | Weight of fat built up in internal organs (g) | Blood Glucose level (mg/dL) |
| Low-fat diet |  |  |  | 27.1 | 0.83 | 183.7 |
| Tapioca starch | Tapioca | - | - | 34.3 | 2.33 | 223.6 |
| Starch octenyl succinate[*1] | Tapioca | octenyl succinated | - | 31.6* | 1.51** | 161.9** |
| Acetylated starch[*2] | Tapioca | Acetylated | - | 30.8** | 1.23*** | 167.2*** |
| Waxy corn starch | Waxy corn | - | - | 33.1 | 1.81 | 220.7 |
| Acetylated starch[*3] | Waxy corn | Acetylated | Adipic acid | 32.5 | 1.75 | 176.8** |
| [*1] Amycol Nyuka, [*2]Z-700, [*3]ADIX-H, and [*4]Mapusu #449 | | | | | | |

The symbol "-" indicates that no chemical treatment was provided to the processed starch.
A statistically significant difference from the corresponding starting material starch:

$$^*P < 0.05, \ ^{**}P < 0.01, \text{ and } ^{***}P < 0.001$$

The results shown in Table 2 show that the mice that ingested the diet wherein 5% of the acetylated starch or the starch octenyl succinate (derived from tapioca) was added thereto showed significantly lower body weight and fat weight of the internal organs than the mouse that ingested the diet wherein the corresponding starting material starch was added thereto, thereby indicating the effect of suppressing obesity.

Further, the mice that ingested the high-fat diet wherein 5% of tapioca starch or waxy corn starch was added thereto showed a higher stationary blood glucose level than the mice that ingested the low-fat diet. However, it is shown that the mice that ingested the high-fat diet wherein 5% of the tapioca starch or the waxy corn starch that was acetylated or modified with octenyl succinate was added thereto showed a lower stationary blood glucose level than the mice that ingested the diet wherein the corresponding starting material starch (derived from tapioca or waxy corn) was added thereto, thereby indicating the effect of suppressing blood glucose level increase.

Test Example 2

The action of suppressing blood glucose level increase after meals exhibited by the acetylated starch or starch octenyl succinate

A tapioca starch and a waxy corn starch were obtained from National Starch and Chemical. As acetylated starch, Z-700 (derived from tapioca, from Nippon Starch Chemical), MT-01B (derived from tapioca, from Nihon Shokuhin Kako), ADIX-H (derived from waxy corn, from Nihon Shokuhin Kako), Mapusu #306 (derived from waxy corn, from Nihon Shokuhin Kako), and Mapusu #449 (derived from waxy corn, from Nihon Shokuhin Kako) were used, and as starch octenyl succinate, Amycol Nyuka (derived from tapioca, from Nippon Starch Chemical) was used.

The above starch (1 g) suspended in 15 mL of distilled water was autoclaved at 120°C for 15 min. (steam treatment), which was allowed to stand to cool down to room temperature.

To the pre-treated starch solution, an alpha-amylase solution, a pepsin solution, a pancreatin solution, and an amyloglucosidase solution were added in this order, and the level of glucose liberated at the end was measured (by Glucose Test Wako, from Wako Pure Chemical).

The above starch was suspended in distilled water at the final concentration of 5% by mass, which then was autoclaved at 120°C for 15 min. (steam treatment). The steam treated solution was allowed to stand to cool down to room temperature. Each group consisted of 6 mice (C57BL/6J, males, 6 weeks old). The above treated starch solution was orally administered to the mice at a dose of 2 mg starch/g body weight. After 0, 30, 60, and 120 min. of administration, the blood samples were collected from the tail vein and the blood glucose level was measured by a compact blood glucose monitoring system (Accu-Chek Comfort from Roche Diagnostics). A blood glucose level increase after the meal was expressed by a percentage of absorption, wherein the area under the blood glucose level curve 2 hrs. after the oral administration of the starting material starch corresponds to 100.

In Table 3, for each of the test starches, the liberated glucose level and the glucose level increase after the meal are shown.

[Table 3]

|  | Derived from | Functional group | Cross-linking | Percentage of digestion (%) calculated from liberated glucose level | Percentage of absorption (%), conventional starch being 100 |
|---|---|---|---|---|---|
| Tapioca starch | Tapioca | - | - | 91.1 | 100 |
| Starch octenyl succinate [*1] | Tapioca | octenyl succinated | - | 87.8*** | 93.2 |
| Acetylated starch[*2] | Tapioca | Acetylated | - | 79.9*** | 77.4* |
| Acetylated starch[*3] | Tapioca | Acetylated | - | 80.1*** | 78.6 |
| Waxy corn starch | Waxy corn | - | - | 92.3*** | 100 |
| Acetylated starch[*4] | Waxy corn | Acetylated | Adipic acid | 75.9*** | 79.5*** |

(continued)

|  | Derived from | Functional group | Cross-linking | Percentage of digestion (%) calculated from liberated glucose level | Percentage of absorption (%), conventional starch being 100 |
|---|---|---|---|---|---|
| Acetylated starch[*5] | Waxy corn | Acetylated | Phosphoric acid | 74.3*** | 73.9** |
| Acetylated starch[*6] | Waxy corn | Acetylated | Phosphoric acid | 76.0*** | 72.6** |
| [*1] Amycol Nyuka, [*2]Z-700, [*3]MT-01B [*4]ADIX-H, [*5]Mapusu #306, and [*6]Mapusu #449 | | | | | |

The symbol "-" indicates that no chemical treatment was provided to the processed starch.
A statistically significant difference from the corresponding starting material starch:

$$^{*}P < 0.05, \ ^{**}P < 0.01, \ \text{and} \ ^{***}P < 0.001$$

The results shown in Table 3 show that the liberated glucose level was significantly lower for the acetylated starch or starch octenyl succinate (derived from tapioca or waxy corn) than for the startingmaterial starch. The blood glucose level increase in the mice to which the acetylated starch (derived from tapioca or waxy corn) was orally administered was also significantly smaller than in the mice to which the corresponding starting material starch was orally administered, thereby indicating the effect of suppressing blood glucose level increase after meals.

## Claims

1.    A preventive/remedy for obesity, wherein acetylated starch or starch octenyl succinate is an active ingredient.

2.    An agent for suppressing fat accumulation in internal organs, wherein acetylated starch or starch octenyl succinate is an active ingredient.

3.    An agent for suppressing blood glucose level increase, wherein acetylated starch or starch octenyl succinate is an active ingredient.

4.    A preventive/remedy for diabetes, wherein acetylated starch or starch octenyl succinate is an active ingredient.

5.    Use of acetylated starch or starch octenyl succinate for preparing a preventive/remedy for obesity.

6.    Use of acetylated starch or starch octenyl succinate for preparing an agent for suppressing fat accumulation in internal organs.

7.    Use of acetylated starch or starch octenyl succinate for preparing an agent for suppressing blood glucose level increase.

8.    Use of acetylated starch or starch octenyl succinate for preparing a preventive/remedy for diabetes.

9.    A method for preventing and ameliorating obesity, wherein acetylated starch or starch octenyl succinate is administered or ingested.

10.    A method for suppressing fat accumulation in internal organs, wherein acetylated starch or starch octenyl succinate is administered or ingested.

**11.** A method for suppressing blood glucose level increase, wherein acetylated starch or starch octenyl succinate is administered or ingested.

**12.** A method for preventing and ameliorating diabetes, wherein acetylated starch or starch octenyl succinate is administered or ingested.

**13.** A food comprising acetylated starch or starch octenyl succinate for preventing and ameliorating obesity.

**14.** A food comprising acetylated starch or starch octenyl succinate for suppressing fat accumulation in internal organs.

**15.** A food comprising acetylated starch or starch octenyl succinate for suppressing blood glucose level increase.

**16.** A food comprising acetylated starch or starch octenyl succinate for preventing and ameliorating diabetes.

# EP 1 788 000 A1

| | | International application No. |
|---|---|---|
| **INTERNATIONAL SEARCH REPORT** | | PCT/JP2005/016402 |

**A.  CLASSIFICATION OF SUBJECT MATTER**

*C08B31/04*(2006.01), *A61K31/718*(2006.01), *A61P1/02*(2006.01), *A61P3/04*(2006.01), *A61P3/06*(2006.01), *A61P3/10*(2006.01), *A61P7/02*(2006.01), *A61P9/04*(2006.01), *A61P9/10*(2006.01), *A61P9/12*(2006.01), *A61P13/12*(2006.01)

According to International Patent Classification (IPC) or to both national classification and IPC

**B.  FIELDS SEARCHED**

Minimum documentation searched (classification system followed by classification symbols)

*C08B31/04*(2006.01), *A61K31/718*(2006.01), *A23L1/30*(2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

CAplus(STN)

**C.  DOCUMENTS CONSIDERED TO BE RELEVANT**

| Category* | Citation of document, with indication, where appropriate, of the relevant passages | Relevant to claim No. |
|---|---|---|
| X | JP 10-279487 A  (Nihon Shokuhin Kako Co., Ltd., Sanwa Cornstarch Co., Ltd.), 20 October, 1998 (20.10.98), Full text (Family: none) | 1-8,13-16 |
| X | JP 2002-534991 A  (COOPERATIEVE VERKOOP- EN PRODUCTIEVERENIGING VAN AARDAPPELMEEL EN DERIVATEN AVEBE B.A.), 22 October, 2002 (22.10.02), Full text & US 6749880 B1          & EP 1146795 A & WO 2000/044241 A1 | 13-16 |

| | |
|---|---|
| ☒  Further documents are listed in the continuation of Box C. | ☐  See patent family annex. |

| | |
|---|---|
| *    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 27 October, 2005 (27.10.05) | 08 November, 2005 (08.11.05) |

| Name and mailing address of the ISA/ | Authorized officer |
|---|---|
| Japanese Patent Office | |
| Facsimile No. | Telephone No. |

Form PCT/ISA/210 (second sheet) (April 2005)

**EP 1 788 000 A1**

| | INTERNATIONAL SEARCH REPORT | International application No. |
|---|---|---|
| | | PCT/JP2005/016402 |

C (Continuation).   DOCUMENTS CONSIDERED TO BE RELEVANT

| Category* | Citation of document, with indication, where appropriate, of the relevant passages | Relevant to claim No. |
|---|---|---|
| X | JP 2002-51756 A  (Nissin Food Products Co., Ltd.),<br>19 February, 2002 (19.02.02),<br>Full text<br>(Family: none) | 13-16 |
| X | JP 10-313804 A  (Nissin Food Products Co., Ltd., Sanwa Cornstarch Co., Ltd.),<br>02 December, 1998 (02.12.98),<br>Full text<br>(Family: none) | 13-16 |
| X | JP 8-242752 A  (Matsutani Chemical Industry Co., Ltd.),<br>24 September, 1996 (24.09.96),<br>Full text<br>(Family: none) | 13-16 |
| X | JP 6-225719 A  (Otsuka Foods Co., Ltd.),<br>16 August, 1994 (16.08.94),<br>Full text<br>& US 5387423 A | 13-16 |
| X | JP 60-164449 A  (National Starch and Chemical Corp.),<br>27 August, 1985 (27.08.85),<br>Full text<br>& JP 6-105652 A          & US 4510166 A<br>& EP 149258 A2          & AU 3697284 A<br>& CA 1226167 A | 13-16 |
| X | JP 4-79861 A  (Matsutani Chemical Industry Co., Ltd.),<br>13 March, 1992 (13.03.92),<br>Full text<br>(Family: none) | 13-16 |
| P,A | WO 2004/080470 A1  (Kao Corp.),<br>23 September, 2004 (23.09.04),<br>Claims<br>& JP 2004-269458 A | 1-8,13-16 |
| A | JP 3-254653 A  (Terumo Corp.),<br>13 November, 1991 (13.11.91),<br>Claims<br>(Family: none) | 1-8,13-16 |

Form PCT/ISA/210 (continuation of second sheet) (April 2005)

## INTERNATIONAL SEARCH REPORT

| International application No. |
|---|
| PCT/JP2005/016402 |

---

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 9-12
   because they relate to subject matter not required to be searched by this Authority, namely:
   The inventions pertain to methods for treatment of the human body by therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:
   There is no technical relationship involving one or more of the same or corresponding special technical features among the inventions according to claims 1 and 5 which relate to an agent for preventing and ameliorating obesity, the inventions according to claims 2 and 6 which relate to an inhibitor for visceral fat accumulation, the inventions according to claims 3 and 7 which relate to an inhibitor for blood glucose level elevation and the inventions according to claims 4 and 8 which relate to an agent for preventing and ameliorating diabetes.  The same applies to the inventions according to claims 1 to 8 and the inventions according to claims 13 to 16.
   Such being the case, the inventions according (continued to extra sheet)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
☐ The additional search fees were accompanied by the applicant's protest and, where applicable, the payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**EP 1 788 000 A1**

**INTERNATIONAL SEARCH REPORT**

| International application No. |
| --- |
| PCT/JP2005/016402 |

Continuation of A. CLASSIFICATION OF SUBJECT MATTER
 (International Patent Classification (IPC))
*A61P25/00*(2006.01), *A61P27/12*(2006.01), *A61P35/00*(2006.01),
*A23L1/30*(2006.01)

(According to International Patent Classification (IPC) or to both national classification and IPC)

Continuation of Box No.III of continuation of first sheet(2)

to claims 1 to 8 and 13 to 16 do not comply with the requirement of unity of invention.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H05186356 A
- JP H03290170 A

**Non-patent literature cited in the description**

- *Am J Clin Nutr,* 1978, vol. 31 (10), 521-529
- *Endocrine Journal,* 1992, vol. 68 (6), 623-35
- *Am JClinNutr,* 1989, vol. 49 (2), 337-44
- *Acta Paediatr Hung,* 1985, vol. 26 (1), 75-7
- *J Endocrinol,* 1995, vol. 144 (3), 533-8
- *Am J Clin Nutr,* 1989, vol. 49 (2), 337-44
- Denpun Seihin No Chishiki. **TOYOHARU TAKAHASHI.** Saiwai Shobo. 112, 132